# EUROPEAN PATENT SPECIFICATION

(11) **EP 3 518 723 B1**
(45) Date of publication and mention of the grant of the patent: **05.10.2022**
(21) Application number: 17777882.6
(22) Date of filing: 29.09.2017
(51) Int. Cl.: A61B 1/00, A61B 1/018, A61M 25/00

(54) **A SET COMPRISING AN ENDOSCOPE AND A WORK TOOL UNIT**
SATZ MIT EINEM ENDOSKOP UND EINER ARBEITSWERKZEUGEINHEIT
ENSEMBLE COMPRENANT UN ENDOSCOPE ET UNE UNITÉ D'OUTIL DE TRAVAIL

(30) Priority: 30.09.2016 DK PA201670777
(43) Date of publication of application: 07.08.2019
(73) Proprietor: Ambu A/S, 2750 Ballerup (DK)
(72) Inventor: JENSEN, Thomas, Bachgaard, 1721 Copenhagen V (DK)
(74) Representative: AWA Denmark A/S
(86) International application number: PCT/EP2017/074752
(87) International publication number: WO 2018/060410

(56) References cited:
- EP-A1- 1 820 457
- EP-A2- 0 813 842
- JP-A- H0 731 587
- US-A1- 2005 070 885
- US-A1- 2015 057 537

## Description

The invention relates to a set comprising a) an endoscope with i) an elongated housing defining an elongated gripping portion for a person to hold around with his hand and ii) an insertion portion with a distal end and b) a work tool unit having a work tool controller and a manipulation tube, such as a coiled wire, with a treatment instrument at the distal end thereof, such as a treatment instrument located near the distal end of the manipulation tube and fixed to the end of an inner cable inside the manipulation tube,

Doctors perform diagnosis and therapy using an endoscope with a flexible insertion portion. The endoscope provides the doctor with the ability to visualize the inside of a patient lumen, and is often designed with an insertion portion with an integral working channel through which a small treatment instrument may be passed in order to, for instance, take samples of tissue or remove an object within the patient body.

Prior art sets of the aforementioned type typically require the use of two hands, one hand to manipulate the insertion portion and another hand to control the treatment instrument within the patient lumen. These existing designs do not allow an endoscopist to operate the set in an ergonomically optimal way.

US patent 7 727 144 discloses a set comprising a) an endoscope with a housing defining a gripping portion and an insertion portion and b) a work tool unit having a work tool controller and a wire with a treatment instrument at the distal end thereof. This prior art set is cumbersome in use in that operating the set requires the operator to use both hands, at least for some functions, and in that rotation of the handle will turn the work tool controller to a position away from the user.

US 2009/0312603 discloses an endoscope with an elongated housing defining an elongated gripping portion having an internal passage and an insertion portion communicating with the internal passage and having a distal end, a work tool unit having a work tool controller and an elongated flexible manipulation tube with a treatment instrument being at the distal end thereof. The housing and the work tool controller have complementary engagement structures for releasably connecting the work tool controller to the housing with the manipulation tube received by the internal passage and the insertion portion. A problem with this device is that the treatment instrument may easily be damaged and that the user cannot control the treatment instrument with his one hand while holding around the gripping portion either with the same hand, or with his other hand, while being able at the same time to rotate the elongated endoscope housing about a longitudinal axis thereof. EP 1 820 457 A1 discloses a set comprising an endoscope with an elongated housing defining an elongated gripping portion having an internal passage and an insertion portion communicating with said internal passage and having a distal end, a work tool unit having a work tool controller and an elongated flexible manipulation tube with a treatment instrument at the distal end thereof, said internal passage extending from a proximal end of said elongated housing and through said elongated gripping portion, said engagement structure of said elongated housing being at said proximal end thereof.

It is an object of the invention to solve the aforementioned problems by providing a set as defined in the characterizing clause of claim 1 wherein the work tool controller is configured for advancing the manipulation tube received by the insertion portion when the work tool controller is connected with the housing from a retracted position to an advanced position relative to the insertion portion, the manipulation tube having an inner cable being connected to the treatment instrument and being movable relative to the manipulation tube.

The endoscopic device of the invention is easy to use in an ergonomically correct way and rotation of the treatment tool for orientation thereof can be performed by an operator holding on to the gripping portion of the endoscope housing. Rotation as mentioned can conveniently be carried out by the operator rotating his hand.

With such a set a user gripping around the elongated gripping portion and rotating it about a longitudinal axis Y of the elongated housing extending between the proximal and distal ends of the housing and defining a major extension of the housing, or an axis essentially parallel therewith, will bring about a corresponding rotation of a work tool unit for orienting and operating the treatment instrument, however without significantly changing the position of the work tool unit relative to the user.

Preferably, the manipulation tube is a coiled wire.

The complementary engagement means are preferably comprised by snap coupling parts or bayonet coupling parts, allowing a quick engagement and disengagement of the work tool unit from the housing.

Preferably the work tool controller is configured for advancing the manipulation tube received by the insertion portion when the work tool controller is connected with said housing at the proximal end thereof, from a retracted position to an advanced position relative to the insertion portion, the treatment instrument being optionally advanced from a position inside the insertion portion to a position projecting from the distal end of the insertion portion when the work tool controller advances the manipulation tube to the advanced position. In this manner, a treatment instrument may be lodged in a protected position inside the insertion portion until use.

In some embodiments the treatment instrument may be a gripper having an opened and a closed position, the gripper being closed or opened when the manipulation tube is in the retracted position. This facilitates the use of treatment instruments having two movable parts, such as a gripper or scissor. Conveniently, the work tool controller may further be configured with a spring an corresponding depressible actuator, or a pair of springs with corresponding depressible actuators. This allows for an even simpler user control of the treatment instrument with a single hand, by allowing the user to apply a pressure onto the actuators, such as in a direction oriented generally along the aforementioned axis Y, or a direction essentially parallel therewith. Preferably, the springs are arranged inside respective housings, even more preferably inside sealed housings, whereby a sterile environment may be achieved.

With an alternative embodiment the work tool controller has a depressible actuator connected to a gear system comprising two interconnected levers, where a first lever is connected to an inner cable as referred to above, and where a second lever is connected to the manipulation tube, optionally with the two levers having lever arms of different length. This may allow for the provision of a particularly user-friendly set allowing the user to control the treatment instrument with his thumb while holding around the gripping portion of the endoscope with the same hand.

In a preferred embodiment, the complementary engagement structures are configured for reengagement after disengagement. This will be of particular advantage where the treatment instrument is to be reinserted into the insertion portion after removal therefrom for inspecting tissue removed from the patient body by a gripper of the treatment instrument.

In the following embodiments of the invention will be described with reference to the schematic figures.
Fig. 1 is a side view of a set according to the invention, comprising *inter alia* an endoscope with an elongated housing, an axis Y defining a major, longitudinal extension thereof,
Fig. 2 is a side view of a work tool unit which is part of the set,
Fig. 3 is a sectional view showing portions of the work tool unit of fig. 2, namely a work tool controller thereof as well as a treatment instrument as the distal end of the work tool unit,
Figs. 4a and 4b are top and partial sectional views, and a side sectional view, showing the endoscope housing and engagement structures for coupling the endoscope housing with the work tool unit, and
Fig. 5 is a view similar to fig. 1 showing another embodiment of the work tool controller in cross-sectional view.

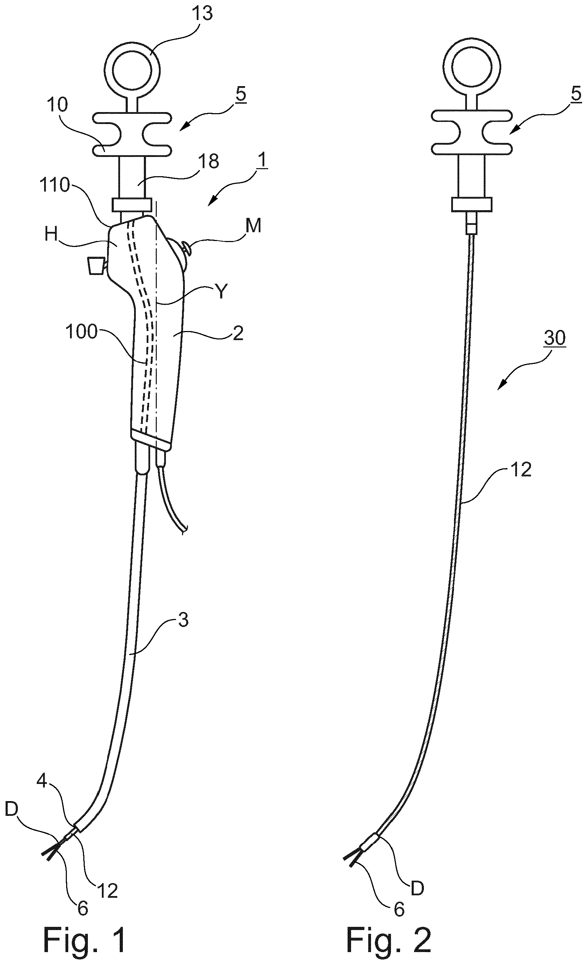

Fig. 1 shows a set according to the invention including an endoscope 1 with i) an elongated housing H having an elongated gripping portion 2 for a user to grip around with his hand and ii) an elongated flexible insertion portion 3 for insertion into a patient, with a distal end 4 including an optical device housing with a camera module and light sources, as shown by way of example in WO 2010/066790.

The insertion portion 3 comprises an elongated internal passage 100 commonly referred to as a working channel extending past the optical device housing to the distal end 4. Often, the insertion portion 3 will include ahead of the optical device housing a bending portion (not shown) for bending the tip of the insertion portion 3, as illustrated by way of example in WO 2014/106511, and being controllable via an actuator M on the housing H.

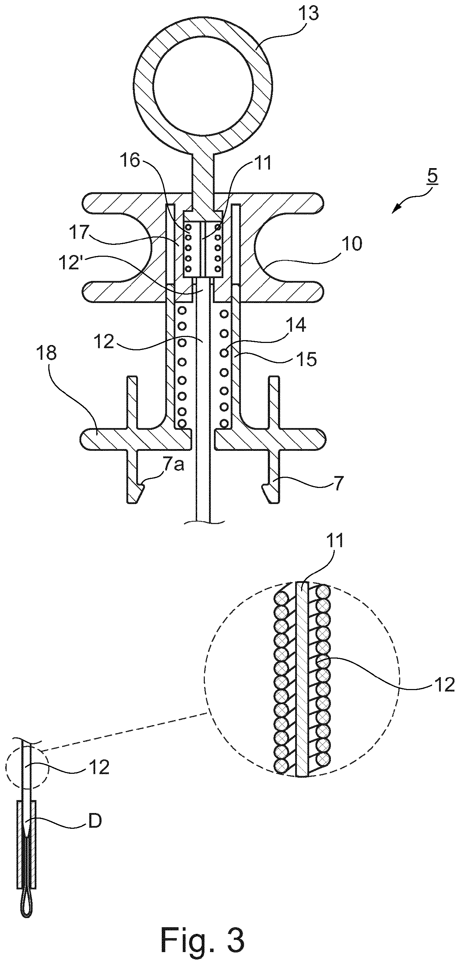

A work tool unit 30, as seen in figs. 2 and 3, has a work tool controller 5 with a base 18 via which the work tool controller 5 is releasably connected with the endoscope 1 in a manner to be described further below. A wire or manipulation tube 12 of the work tool unit 30 has in fig. 1 been inserted into the insertion portion 3, via a portion of the internal passage 100 extending in the endoscope 1 housing H.

Specifically, the internal passage 100 extends from a proximal end 110 of the elongated housing H and through the gripping portion 2, an engagement structure part 8 of the housing H being at the proximal end 110 and discussed further below, and the insertion portion 3 extends from the distal end of the housing H, opposite the proximal end 110 of the elongated housing H. A longitudinal axis Y of the elongated housing H, defining a major extension thereof, extends between the proximal end 110 and distal end of the elongated housing H.

The work tool unit 30 shown in fig. 2 comprises the work tool controller 5 and the manipulation tube 12 with a treatment instrument 6 at the distal end thereof. In the shown embodiment is this treatment instrument a gripper 6 or a scissor. However the invention is not limited to any particular type of treatment instrument.

The length of the manipulation tube 12 from the work tool controller 5 is selected to correspond to the length of the internal passage, from the proximal end 110 of the housing to the distal end 4 of the insertion portion 3, such that the treatment instrument mounted to the end of an inner cable 11 does not project from the distal end 4 of the insertion portion 3, until operated to do so.

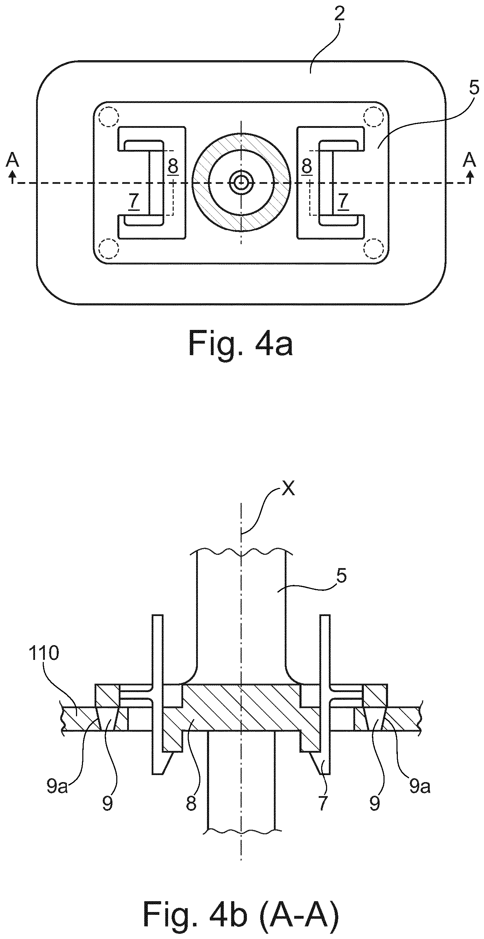

Fig. 3 illustrates an embodiment of the work tool controller 5 in details, showing in particular an engagement structure part 7 integral with the base 18 of the work tool controller 5 and configured for being releasably connected with the aforementioned complementary engagement structure part 8 arranged on or integral with the housing H, as shown in figs. 4a and 4b, wherein the work tool controller 5 is releasably connected with the housing H, at the proximal end 110 of the latter. Specifically, the engagement structure part 7 arranged on the base 18 of the work tool unit 5 comprises two resilient legs 7 with hook portions 7a engaging respective apertures 8 formed in the housing H, the apertures 8 being sized for narrowly receiving the legs 7 and configured such that the hook portion 7a may bear against an inside surface portion of the housing H. The legs 7 have such a length that a person may apply a force onto the end of the legs 7 opposite the hook portions 7a, thereby deflecting the legs 7 and, hence, bringing the hook portion 7a out of engagement with the housing H to allow for removal of the work tool controller 5 from the housing H. This narrow or tight engagement ensures that the work tool controller 5 essentially cannot be rotated relative to the housing H, about an axis shown by letter X in fig. 4b; allowing for some play or tolerances the rotation is prevented or restricted.

The engagement structures, in the shown embodiment, also include guide pins 9 provided on the base 18 of the work tool controller 5. The guide pins 9 are received in complementary apertures 9a formed in the housing H.

The work tool unit 5 further is shown with a depressible first actuator 10 to which the end 12' of the manipulation tube 12 is fixedly anchored for advancing and retracting of the flexible manipulation tube 12 relative to the insertion portion 3; the anchoring is such that twisting or rotation of the manipulation tube 12 about its longitudinal axis relative to the actuator 12 is prevented. The depressible first actuator 10 is slidably connected to the base 18 having the engagement structures 7, 7a, via a first housing 15 on the base 18, in which housing 15 is lodged a spring 14 which pushes the first actuator 10 away from the base 18 and thereby holds the flexible manipulation tube 12 biased towards the aforementioned retracted position. Rotation between the first actuator 10 and the housing 15 and, hence, the base 18 is prevented or restricted. In use the first and second actuators 10, 12 are displaced along the direction identified generally by letter X in fig. 4b.

By this design and by having an internal passage 100 defined by an inner tube inside the insertion portion 3 which runs to the proximal end 110 of the elongated housing H it is achieved that rotation of the endoscope 1, by the user gripping with his hand around the elongated gripping portion 2, about the longitudinal axis Y of the elongated housing H, or an axis essentially parallel therewith, brings about a corresponding rotation of the work tool unit 30, however without changing the position of the work tool unit 30 relative to the user.

The work tool controller 5 has a second actuator 13 for controlling the inner cable 11, such as for rotating the inner cable 11 relative to said manipulation tube 12 and/or for advancing said inner cable 11 along the length of said manipulation tube 12. The second actuator 13 is connected to the first actuator 10 via a second housing 17 provided internally in the first actuator 10 and the second housing 17 is internally provided with a compression spring 14 which pulls the inner cable 11 towards a retracted position. By rotating the second actuator, the treatment instrument 6 (not shown in figure 4) may, in embodiments of the invention, be rotated relatively to the manipulation tube 12.

Preferably the first actuator 10, the second actuator 13 and the base 18 are three injection moulded parts configured for being assembled to form the work tool controller 5.

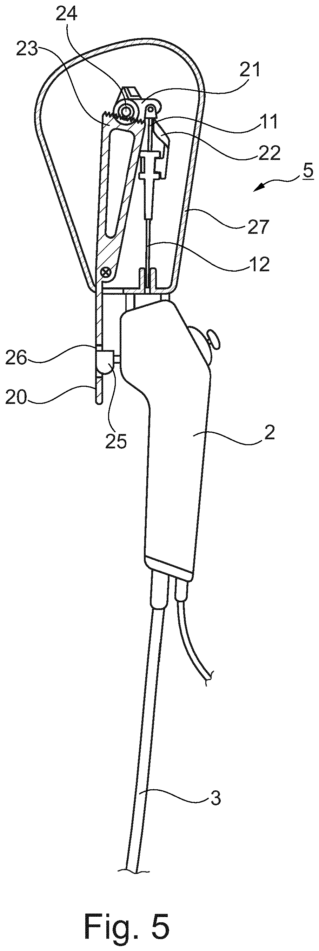

In fig. 5 is shown an alternative embodiment of a work tool controller 5 for operation of the manipulation tube 12 and the inner cable 11. The work tool controller 5, in this embodiment, is provided with a handle 20 defining a third type actuator and being connected to a gear system 23, 24 linked with a first and a second lever 21, 22 being connected to the inner cable 11 and the manipulation tube 12 respectively. The handle 20 is preferably spring assisted (not shown) such that it returns to an original state when not depressed. The first and second levers 21, 22 have different lengths and each length is adapted such that one length of the manipulation tube 12 and another length of the inner cable 11 is either advanced or retracted relative to the distal end 4 of the insertion portion 3, when the work tool controller handle 20 is moved (e.g. depressed or released). Preferably the length of the levers is adapted such that movement of the handle 20 causes the inner cable to activate the treatment instrument 6 (e.g. open or close it) while at the same time the manipulation tube 12 is retracted or extended. The depression of the handle 20 converts a continuous movement of the manipulation tube 12 and the inner cable 11 into a compound movement in which a first part of said continuous movement effects the linear movement of the manipulation tube 12, and at least one second part of said continuous movement effects the activation of the treatment instrument 6 by moving the inner cable 11. The handle or depressible actuator 20 may be provided with an opening 26 such that a bottom 25 on the endoscope can be manipulated through the opening. Preferably the lengths of the levers 21, 22, the internal passage 100 (not shown in figure 5), the manipulation tube 12 and the inner cable 11 are mutually adapted such that operation of the handle causes the treatment instrument to be activated (e.g. opened or close) while the manipulation tube is either retracted or extended from the insertion portion 3. The providing of a set comprising engagement structures 7,8, levers 21, 22, gear system 23, 24, and adapted lengths as described above entails that an operator can manipulate an insertion portion 3 of an endoscope 1 through a body lumen, and extend the manipulation tube 12 from a retracted position within the insertion portion 3 to an extended position outside the insertion portion 3 and further active and treatment instrument 6 with the use of a single hand. By providing the handle 20 with an opening 26 as described above further entails that e.g. suction or flushing can be added as a single hand operation to the above already mentioned single hand operations.

It is noted that by enclosing - by the aforementioned dedicated housings 15, 17, 27 - the levers/gear system 23, 24, springs 14, 16, portion of the manipulation tube 12 and the inner cable 11, as shown in figs. 3 and 5 allows for obtaining a sterile environment.

The manipulation tube 12 may, in any embodiment of the invention, be made of coiled plastic/metal wire or it can be a plastic tube or any other material known to the skilled person and suitable therefore.

## Claims

1. A set comprising:
- an endoscope (1) with
- - i) an elongated housing (H) defining an elongated gripping portion (2) having an internal passage (100) and
- - ii) an insertion portion (3) communicating with said internal passage (100) and having a distal end (4),
- a work tool unit (30) having a work tool controller (5) and an elongated flexible manipulation tube (12) with a treatment instrument (6) at the distal end (D) thereof,
- said housing (H) and said work tool controller (5) having complementary engagement structures (7, 8) for releasably connecting said work tool controller (5) to said housing (H) with said manipulation tube (12) received by said internal passage (100) and said insertion portion (3),
- said internal passage (100) extending from a proximal end (110) of said elongated housing (H) and through said elongated gripping portion (2), said engagement structure (8) of said elongated housing (H) being at said proximal end (110) thereof,
wherein
- said engagement structures (7, 8) being configured for preventing rotation of at least a base (18) of said work tool controller (5) relative to said housing (H) releasably connected with said work tool controller (5),
- wherein free rotation of said manipulation tube (12) relative to said base (18) is prevented,
- said work tool controller (5) being configured for advancing said manipulation tube (12) received by said insertion portion (3) when said work tool controller (5) is connected with said housing (H) from a retracted position to an advanced position relative to said insertion portion (3),
- said manipulation tube (12) having an inner cable (11), said inner cable (11) being connected to said treatment instrument (6) and being movable relative to said manipulation tube (12).

2. A set according to claim 1, said complementary engagement structures (7, 8) being male and female snap coupling parts, respectively.

3. A set according to claim 1 or 2, said complementary engagement structures (7, 8) being bayonet coupling parts.

4. A set according to any of the previous claims, said treatment instrument (6) being advanced from a position inside said insertion portion (3) to a position projecting from said distal end (4) of said insertion portion (3) on said work tool controller (5) advancing said manipulation tube (12) to said advanced position.

5. A set according to any of the previous claims, said treatment instrument (6) being activated when said inner cable (11) is moved relative to said manipulation tube (12).

6. A set according to any of the previous claims, said treatment instrument being a gripper (6) having an opened and a closed position, said gripper (6) being closed or opened when said manipulation tube (12) is in said retracted position.

7. A set according to any of the previous claims when dependent on claim 4, a first spring (14) biasing said manipulation tube (12) towards said retracted position.

8. A set according to any of the previous claims when dependent on claim 4, said work tool controller (5) having a depressible first actuator (10) for said advancing of said manipulation tube (12)..

9. A set according to any of the previous claims, when dependent on claim 4, said work tool controller (5) having a second actuator (13) for controlling said inner cable (11), such as for rotating said inner cable (11) relative to said manipulation tube (12) and/or for advancing said inner cable (11) along the length of said manipulation tube (12).

10. A set according to claim 9 when dependent of claim 8, said work tool controller (5) comprising a first housing (15) enclosing a portion of said manipulation tube (12) and said first spring (14), said first actuator (10) being movably connected to said first housing (15).

11. A set according to claim 10, a second spring (16) biasing said inner cable (11) towards a retracted position relative to said manipulation tube (12).

12. A set according to claim 11, said first actuator (10) comprising a second housing (17) enclosing a portion of said inner cable (11) and said second spring (16), said second actuator (13) being moveably connected to said second housing (17).

13. A set according to any of the previous claims, said work tool controller (5) having a depressible actuator (20) being connected to a gear system comprising two interconnected levers, a first lever (21) being connected to said inner cable (11) and a second lever (22) being connected to said manipulation tube (12).

14. A set according to claim 13, said first and second levers (21, 22) having lever arms of different length.

15. A set according to any of the previous claims, said complementary engagement structures (7, 8) being configured for reengagement after disengagement.

## Patentansprüche

1. Satz, umfassend:
- ein Endoskop (1) mit
-- i) einem länglichen Gehäuse (H), das einen länglichen Greifabschnitt (2) mit einem inneren Durchgang (100) definiert, und
-- ii) einem Einführabschnitt (3), der mit dem inneren Durchgang (100) in Verbindung steht und ein distales Ende (4) hat,
- eine Arbeitswerkzeugeinheit (30) mit einer Arbeitswerkzeugsteuerung (5) und einer länglichen flexiblen Handhabungsröhre (12) mit einem Behandlungsinstrument (6) an dem distalen Ende (D) davon,
- wobei das Gehäuse (H) und die Arbeitswerkzeugsteuerung (5) komplementäre Eingriffsstrukturen (7, 8) haben, um die Arbeitswerkzeugsteuerung (5) freigebbar mit dem Gehäuse (H) zu verbinden, wobei die Handhabungsröhre (12) von dem inneren Durchgang (100) und dem Einführabschnitt (3) aufgenommen wird,
- wobei sich der innere Durchgang (100) von einem proximalen Ende (110) des länglichen Gehäuses (H) und durch den länglichen Greifabschnitt (2) erstreckt, wobei die Eingriffsstruktur (8) des länglichen Gehäuses (H) an dem proximalen Ende (110) davon ist,
wobei
- die Eingriffsstrukturen (7, 8) dazu ausgestaltet sind, eine Drehung mindestens einer Basis (18) der Arbeitswerkzeugsteuerung (5) bezüglich des freigebbar mit der Arbeitswerkzeugsteuerung (5) verbundenen Gehäuses (H) zu verhindern,
- wobei eine freie Drehung der Handhabungsröhre (12) bezüglich der Basis (18) verhindert wird,
- wobei die Arbeitswerkzeugsteuerung (5) dazu ausgestaltet ist, die von dem Einführabschnitt (3) aufgenommene Handhabungsröhre (12) aus einer zurückgezogenen Position in eine vorgeschobene Position bezüglich des Einführabschnitts (3) vorzuschieben, wenn die Arbeitswerkzeugsteuerung (5) mit dem Gehäuse (H) verbunden ist,
- wobei die Handhabungsröhre (12) ein inneres Kabel (11) hat, wobei das innere Kabel (11) mit dem Behandlungsinstrument (6) verbunden und bezüglich der Handhabungsröhre (12) beweglich ist.

2. Satz nach Anspruch 1, wobei die komplementären Eingriffsstrukturen (7, 8) männliche bzw. weibliche Einschnappkopplungsteile sind.

3. Satz nach Anspruch 1 oder 2, wobei die komplementären Eingriffsstrukturen (7, 8) Bajonettkopplungsteile sind.

4. Satz nach einem der vorhergehenden Ansprüche, wobei das Behandlungsinstrument (6) von einer Position in dem Einführabschnitt (3) in eine Position vorgeschoben wird, in der es von dem distalen Ende (4) des Einführabschnitts (3) vorragt, wenn die Arbeitswerkzeugsteuerung (5) die Handhabungsröhre (12) zu der vorgeschobenen Position vorschiebt.

5. Satz nach einem der vorhergehenden Ansprüche, wobei das Behandlungsinstrument (6) aktiviert wird, wenn das innere Kabel (11) bezüglich der Handhabungsröhre (12) bewegt wird.

6. Satz nach einem der vorhergehenden Ansprüche, wobei das Behandlungsinstrument ein Greifer (6) mit einer geöffneten und einer geschlossenen Position ist, wobei der Greifer (6) geschlossen oder geöffnet wird, wenn die Handhabungsröhre (12) in der zurückgezogenen Position ist.

7. Satz nach einem der vorhergehenden Ansprüche, wenn von Anspruch 4 abhängig, wobei eine erste Feder (14) die Handhabungsröhre (12) zu der zurückgezogenen Position hin vorspannt.

8. Satz nach einem der vorhergehenden Ansprüche, wenn von Anspruch 4 abhängig, wobei die Arbeitswerkzeugsteuerung (5) einen niederdrückbaren ersten Aktuator (10) zum Vorschieben der Handhabungsröhre (12) hat.

9. Satz nach einem der vorhergehenden Ansprüche, wenn von Anspruch 4 abhängig, wobei die Arbeitswerkzeugsteuerung (5) einen zweiten Aktuator (13) zum Steuern des inneren Kabels (11) wie zum Drehen des inneren Kabels (11) bezüglich der Handhabungsröhre (12) und/oder zum Vorschieben des inneren Kabels (11) entlang der Länge der Handhabungsröhre (12) hat.

10. Satz nach Anspruch 9, wenn von Anspruch 8 abhängig, wobei die Arbeitswerkzeugsteuerung (5) ein erstes Gehäuse (15) umfasst, das einen Abschnitt der Handhabungsröhre (12) und die erste Feder (14) umschließt, wobei der erste Aktuator (10) beweglich mit dem ersten Gehäuse (15) verbunden ist.

11. Satz nach Anspruch 10, wobei eine zweite Feder (16) das innere Kabel (11) zu einer zurückgezogenen Position bezüglich der Handhabungsröhre (12) hin vorspannt.

12. Satz nach Anspruch 11, wobei der erste Aktuator (10) ein zweites Gehäuse (17) umfasst, das einen Abschnitt des inneren Kabels (11) und die zweite Feder (16) umschließt, wobei der zweite Aktuator (13) beweglich mit dem zweiten Gehäuse (17) verbunden ist.

13. Satz nach einem der vorhergehenden Ansprüche, wobei die Arbeitswerkzeugsteuerung (5) einen niederdrückbaren Aktuator (20) hat, der mit einem Getriebesystem verbunden ist, das zwei miteinander verbundene Hebel umfasst, wobei ein erster Hebel (21) mit dem inneren Kabel (11) verbunden ist und ein zweiter Hebel (22) mit der Handhabungsröhre (12) verbunden ist.

14. Satz nach Anspruch 13, wobei der erste und der zweite Hebel (21, 22) Hebelarme unterschiedlicher Länge haben.

15. Satz nach einem der vorhergehenden Ansprüche, wobei die komplementären Eingriffsstrukturen (7, 8) für den Wiedereingriff nach dem Ausrücken ausgestaltet sind.

## Revendications

1. Ensemble comprenant :
- un endoscope (1) avec
-- i) un boîtier allongé (H) définissant une partie de préhension allongée (2) ayant un passage interne (100) et
-- ii) une partie d'insertion (3) communiquant avec ledit passage interne (100) et ayant une extrémité distale (4),
- une unité d'outil de travail (30) ayant un dispositif de commande d'outil de travail (5) et un tube de manipulation flexible allongé (12) avec un instrument de traitement (6) à son extrémité distale (D),
- ledit boîtier (H) et ledit dispositif de commande d'outil de travail (5) ayant des structures de mise en prise complémentaires (7, 8) pour relier de manière amovible ledit dispositif de commande d'outil de travail (5) audit boîtier (H) avec ledit tube de manipulation (12) reçu par ledit passage interne (100) et ladite partie d'insertion (3),
- ledit passage interne (100) s'étendant depuis une extrémité proximale (110) dudit boîtier allongé (H) et à travers ladite partie de préhension allongée (2), ladite structure de mise en prise (8) dudit boîtier allongé (H) se trouvant à ladite extrémité proximale (110) de celui-ci,
- lesdites structures de mise en prise (7, 8) étant configurées pour empêcher la rotation d'au moins une base (18) dudit dispositif de commande d'outil de travail (5) par rapport audit boîtier (H) relié de manière amovible audit dispositif de commande d'outil de travail (5),
- la rotation libre dudit tube de manipulation (12) par rapport à ladite base (18) étant empêchée,
- ledit dispositif de commande d'outil de travail (5) étant configuré pour faire avancer ledit tube de manipulation (12) reçu par ladite partie d'insertion (3) lorsque ledit dispositif de commande d'outil de travail (5) est relié audit boîtier (H), d'une position rétractée à une position avancée par rapport à ladite partie d'insertion (3),
- ledit tube de manipulation (12) ayant un câble intérieur (11), ledit câble intérieur (11) étant relié audit instrument de traitement (6) et étant mobile par rapport audit tube de manipulation (12).

2. Ensemble selon la revendication 1, lesdites structures de mise en prise complémentaires (7, 8) étant des pièces d'accouplement à enclenchement mâle et femelle, respectivement.

3. Ensemble selon la revendication 1 ou 2, lesdites structures de mise en prise complémentaires (7, 8) étant des pièces d'accouplement à baïonnette.

4. Ensemble selon l'une quelconque des revendications précédentes, ledit instrument de traitement (6) étant avancé depuis une position à l'intérieur de ladite partie d'insertion (3) jusqu'à une position faisant saillie depuis ladite extrémité distale (4) de ladite partie d'insertion (3) sur ledit dispositif de commande d'outil de travail (5) faisant avancer ledit tube de manipulation (12) jusqu'à ladite position avancée.

5. Ensemble selon l'une quelconque des revendications précédentes, ledit instrument de traitement (6) étant activé lorsque ledit câble interne (11) est déplacé par rapport audit tube de manipulation (12).

6. Ensemble selon l'une quelconque des revendications précédentes, ledit instrument de traitement étant un dispositif de préhension (6) ayant une position ouverte et une position fermée, ledit dispositif de préhension (6) étant fermé ou ouvert lorsque ledit tube de manipulation (12) est dans ladite position rétractée.

7. Ensemble selon l'une quelconque des revendications précédentes, lorsqu'elle dépend de la revendication 4, un premier ressort (14) sollicitant ledit tube de manipulation (12) vers ladite position rétractée.

8. Ensemble selon l'une quelconque des revendications précédentes lorsqu'elle dépend de la revendication 4, ledit dispositif de commande d'outil de travail (5) ayant un premier actionneur (10) pouvant être enfoncé pour ladite avancée dudit tube de manipulation (12).

9. Ensemble selon l'une quelconque des revendications précédentes, lorsqu'elle dépend de la revendication 4, ledit dispositif de commande d'outil de travail (5) ayant un second actionneur (13) pour commander ledit câble interne (11), tel que pour faire tourner ledit câble interne (11) par rapport audit tube de manipulation (12) et/ou pour faire avancer ledit câble interne (11) sur la longueur dudit tube de manipulation (12).

10. Ensemble selon la revendication 9 lorsqu'elle dépend de la revendication 8, ledit dispositif de commande d'outil de travail (5) comprenant un premier boîtier (15) enfermant une partie dudit tube de manipulation (12) et dudit premier ressort (14), ledit premier actionneur (10) étant relié de manière mobile audit premier boîtier (15).

11. Ensemble selon la revendication 10, un second ressort (16) sollicitant ledit câble interne (11) vers une position rétractée par rapport audit tube de manipulation (12).

12. Ensemble selon la revendication 11, ledit premier actionneur (10) comprenant un second boîtier (17) enfermant une partie dudit câble interne (11) et dudit second ressort (16), ledit second actionneur (13) étant relié de manière mobile audit second boîtier (17).

13. Ensemble selon l'une quelconque des revendications précédentes, ledit dispositif de commande d'outil de travail (5) ayant un actionneur pouvant être enfoncé (20) étant relié à un système d'engrenage comprenant deux leviers interconnectés, un premier levier (21) étant relié audit câble interne (11) et un second levier (22) étant relié audit tube de manipulation (12).

14. Ensemble selon la revendication 13, lesdits premier et second leviers (21, 22) ayant des bras de levier de longueur différente.

15. Ensemble selon l'une quelconque des revendications précédentes, lesdites structures de mise en prise complémentaires (7, 8) étant configurées pour un remise en prise après libération.
